# EUROPEAN PATENT SPECIFICATION

(11) **EP 3 132 054 B1**
(45) Date of publication and mention of the grant of the patent: **30.06.2021**
(21) Application number: 15719948.0
(22) Date of filing: 17.04.2015
(51) Int. Cl.: C12Q 1/68

(54) **MET FUSIONS**
MET-FUSIONEN
FUSIONS DE MET

(30) Priority: 18.04.2014 US 201461981537 P
(43) Date of publication of application: 22.02.2017
(73) Proprietor: Blueprint Medicines Corporation, Cambridge, MA 02139 (US)
(72) Inventor: CERAMI, Ethan, G., Winchester, MA 01890 (US); LENGAUER, Christoph, Cambridge, MA 02129 (US); STRANSKY, Nicolas, Charlestown, MA 02129 (US)
(74) Representative: Finnegan Europe LLP
(86) International application number: PCT/US2015/026505
(87) International publication number: WO 2015/161277

(56) References cited:
- WO-A1-2014/039971
- WO-A2-2012/092336
- NEELA R SOMAN ET AL: "The TPR-MET oncogenic rearrangement is present and expressed in human gastric carcinoma and precursor lesions.", PROC NATL ACAD SCI USA, vol. 88, 1 June 1991 (1991-06-01), pages 4892-4896, XP55203659,
- NEELA R SOMAN ET AL: "TPR-MET oncogenic rearrangement: detection by polymerase chain reaction amplification of the transcript and expression in human tumor cell lines", PROC NATL ACAD SCI USA, vol. 87, 1 January 1990 (1990-01-01), pages 738-742, XP055203647,
- SCHMIDT LAURA ET AL: "Germline and somatic mutations in the tyrosine kinase domain of the MET proto-oncogene in papillary renal carcinomas", NATURE GENETICS, NATURE PUBLISHING GROUP, NEW YORK, US, vol. 16, no. 1, 16 May 1997 (1997-05-16), pages 68-73, XP002557213, ISSN: 1061-4036, DOI: 10.1038/NG0597-68
- MA P C ET AL: "Functional expression and mutations of c-met and its therapeutic inhibition with SU11274 and small interfering RNA in non-small cell lung cancer", CANCER RESEARCH, AMERICAN ASSOCIATION FOR CANCER RESEARCH, US, vol. 65, no. 4, 15 February 2005 (2005-02-15), pages 1479-1488, XP002384470, ISSN: 0008-5472, DOI: 10.1158/0008-5472.CAN-04-2650
- H H L MAK ET AL: "Oncogenic activation of the Met receptor tyrosine kinase fusion protein, Tpr-Met, involves exclusion from the endocytic degradative pathway", ONCOGENE, vol. 26, no. 51, 28 May 2007 (2007-05-28), pages 7213-7221, XP055203148, ISSN: 0950-9232, DOI: 10.1038/sj.onc.1210522
- NICOLAS STRANSKY ET AL: "The landscape of kinase fusions in cancer", NATURE COMMUNICATIONS, vol. 5, 10 September 2014 (2014-09-10), page 4846, XP055223951, DOI: 10.1038/ncomms5846
- SEBASTIAN BENDER ET AL: "Recurrent MET fusion genes represent a drug target in pediatric glioblastoma", NATURE MEDICINE, vol. 22, no. 11, 17 November 2016 (2016-11-17), pages 1314-1320, XP055575640, New York ISSN: 1078-8956, DOI: 10.1038/nm.4204

## Description

This invention relates to MET gene fusions and MET fusion proteins. The invention further relates to methods of diagnosing and treating diseases or disorders associated with MET fusions, such as conditions mediated by aberrant MET expression or activity, or conditions associated with overexpression of MET.

Many forms of cancer are caused by genetic lesions that give rise to tumor initiation and growth. Genetic lesions may include chromosomal aberrations, such as translocations, inversions, deletions, copy number changes, gene expression level changes, and somatic and germline mutations.

Indeed, the presence of such genomic aberrations is a hallmark feature of many cancers, including, for example, B cell cancer, lung cancer, breast cancer, ovarian cancer, pancreatic cancer, and colon cancer. In some models, cancer represents the phenotypic endpoint of multiple genetic lesions that endow cells with a full range of biological properties required for tumorigenesis.

Recent efforts by The Cancer Genome Atlas (TCGA), the International Cancer Genome Consortium (ICGC), and dozens of other large-scale profiling efforts have generated an enormous amount of new sequencing data for dozens of cancer types - this includes wholegenome DNA, whole-exome DNA, and full-transcriptome RNA sequencing. These efforts have led to the identification of new driver genes and fusion genes within multiple cancer types. Fusions, particularly fusions involving kinases, are of particular interest, as such fusions have been shown to be oncogenic, and have been successfully targeted by new therapeutics. For example, anaplastic lymphoma kinase (ALK), one of the receptor tyrosine kinases, is known to become oncogenic when fused with various genes. See, e.g., M. Soda et al, "Identification of the transforming EML4-ALK fusion gene in non-small-cell lung cancer," Nature 444:561-566 (2007).

A need exists for identifying novel genetic lesions associated with cancer. For example, the presence of fusions involving a kinase in samples collected from more than one source can indicate that the kinase is an oncogenic driver. The identification of such fusions can be an effective approach to diagnosis of cancers and development of compounds, compositions, methods, and assays for evaluating and treating cancer patients.

In one aspect, the invention provides methods for detecting the presence of a MET fusion in a biological sample; the methods include the steps of contacting the sample with a reagent that detects a MET fusion, to determine whether a MET fusion is present in the biological sample, in accordance with the claims. In some embodiments, the sample can be from, e.g., a cancer patient. In some embodiments, the cancer is carcinoma of the kidney, brain, liver, lung, or thyroid. In some embodiments, the cancer is selected from papillary renal cell carcinoma, brain lower grade glioma, hepatocellular carcinoma, lung adenocarcinoma, and thyroid carcinoma. In some embodiments, the fusion can be, e.g., a BAIAP2L1:MET fusion, a C8orf34:MET fusion, a PTPRZ1:MET fusion, an OXR1:MET fusion, a KIF5B:MET fusion, or a TFG:MET fusion. In some embodiments, the BAIAP2L1:MET fusion has all or a part of the nucleotide and/or amino acid sequence (such as, e.g., the fusion junction) set forth in SEQ ID NO:1 and SEQ ID NO:2, respectively. In some embodiments, the C8orf34:MET fusion has all or part of the nucleotide and/or amino acid sequence (such as, e.g., the fusion junction) set forth in SEQ ID NO:3 and SEQ ID NO:4, respectively. In some embodiments, the PTPRZ1:MET fusion has all or part of the nucleotide and/or amino acid sequence (such as, e.g., the fusion junction) set forth in SEQ ID NO:5 and SEQ ID NO:6, respectively. In some embodiments, the OXR1:MET fusion has all or part of the nucleotide and/or amino acid sequence (such as, e.g., the fusion junction) set forth in SEQ ID NO:7 and SEQ ID NO:8, respectively. In some embodiments, the OXR1:MET fusion has all or part of the nucleotide and/or amino acid sequence (such as, e.g., the fusion junction) set forth in SEQ ID NO:9 and SEQ ID NO:10, respectively. In some embodiments, the KIF5B:MET fusion has all or part of the nucleotide and/or amino acid sequence (such as, e.g., the fusion junction) set forth in SEQ ID NO:11 and SEQ ID NO:12, respectively. In some embodiments, the TFG:MET fusion has all or part of the nucleotide and/or amino acid sequence (such as, e.g., the fusion junction) set forth in SEQ ID NO:13 and SEQ ID NO:14, respectively.

In another aspect, the invention provides methods of diagnosing a patient having cancer the methods include contacting the sample with a reagent that detects a MET fusion to determine whether a MET fusion is present in the biological sample, wherein the detection of the MET fusion indicates the presence of or susceptibility to cancer, in accordance with the claims.

The invention also a molecule capable of specifically binding to a BAIAP2L1:MET, C8orf34:MET, PTPRZ1:MET, OXR1:MET, KIF5B:MET, or TFG:MET gene fusion, wherein the molecule is an oligonucleotide that hybridizes to the fusion junction of a MET gene fusion selected from BAIAP2L1:MET, C8orf34:MET, PTPRZ1:MET, OXR1:MET, KIF5B:MET, and TFG:MET, in accordance with the claims.

### BRIEF DESCRIPTION OF THE FIGURES

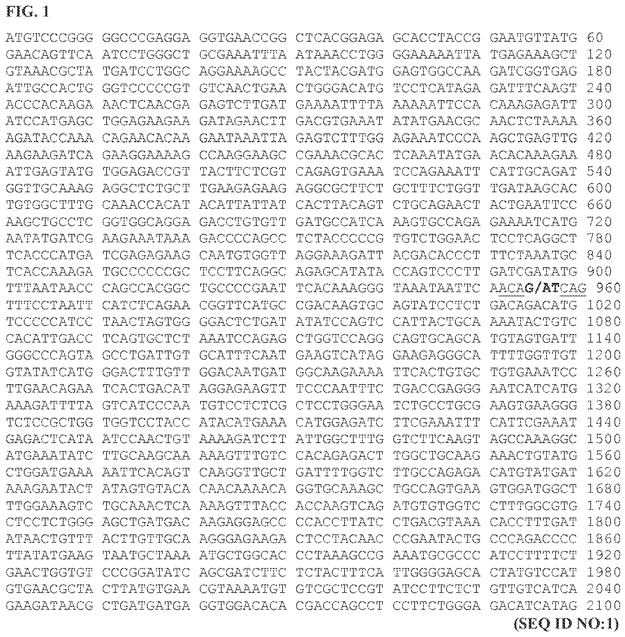

**FIG. 1** depict the nucleotide sequence of a BAIAP2L1:MET gene fusion (SEQ ID NO:1) comprising a portion of the Brain-specific angiogenesis inhibitor 1-associated protein 2-like protein 1 (BAIAP2L1) gene (NM_018842) and a portion of the MET gene (NM_000245). The underlined codons at nucleotides 952-954 and 958-960 encode the last amino acid of BAIAP2L1 and the first amino acid of MET, respectively. The slash after nucleotide 955 indicates the breakpoint (fusion junction) where translocation and in-frame fusion has occurred. The shading at nucleotides 955-957 indicates that nucleotides from both BAIAP2L1 and MET are fused in frame to form a codon and encode an amino acid.

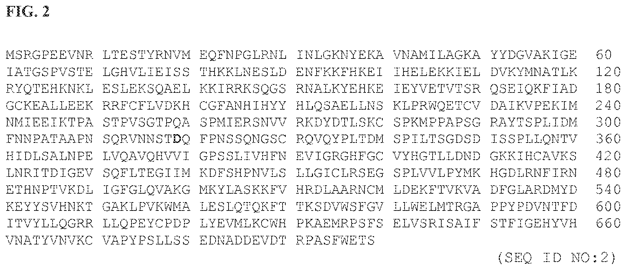

**FIG. 2** depicts the amino acid sequence of a BAIAP2L1:MET fusion protein (SEQ ID NO:2). The amino acid in bold at position 319 corresponds to nucleotides 955-957 in SEQ ID NO:1. This amino acid is encoded by nucleotides from both BAIAP2L1 and MET.

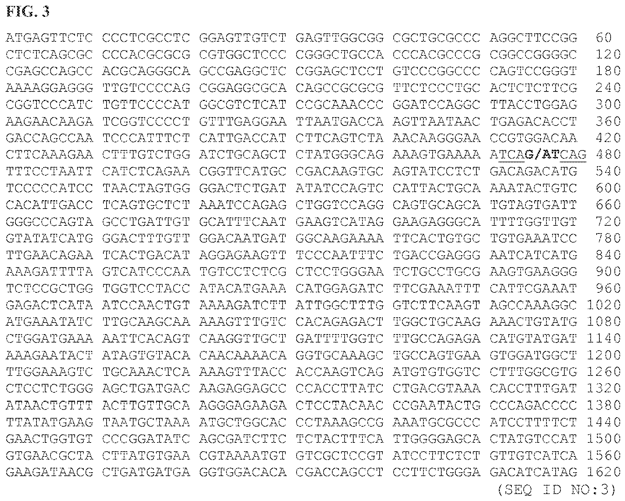

**FIG. 3** depicts the nucleotide sequence of a C8orf34:MET gene fusion (SEQ ID NO:2) comprising a portion of the chromosome 8 open reading frame 34 (C8orf34) gene (NM_052958) and a portion of the MET gene (NM_000245). The underlined codons at nucleotides 472-474 and 478-480 encode the last amino acid of C8orf34 and the first amino acid of MET, respectively. The slash after nucleotide 475 indicates the breakpoint (fusion junction) where translocation and in-frame fusion has occurred. The shading at nucleotides 475-477 indicates that nucleotides from both C8orf34 and MET are fused in frame to form a codon and encode an amino acid.

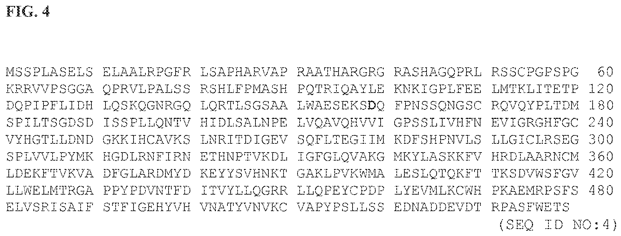

**FIG. 4** depicts the amino acid sequence of a C8orf34:MET fusion protein (SEQ ID NO:4). The amino acid in bold at position 159 corresponds to nucleotides 475-477 in SEQ ID NO:2. This amino acid is encoded by nucleotides from both C8orf34 and MET.

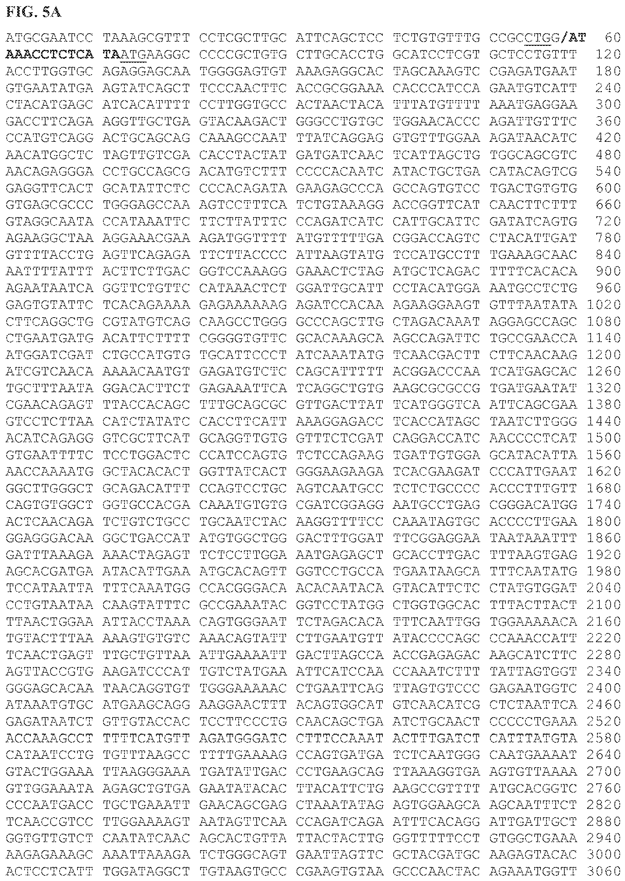

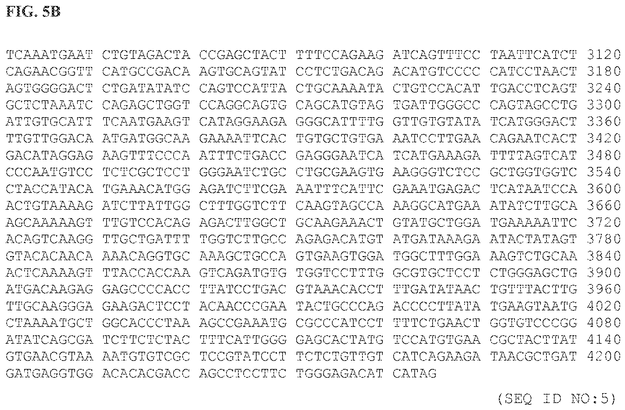

**FIG. 5A** **&** **5B** depict the nucleotide sequence of a PTPRZ1:MET gene fusion (SEQ ID NO:5) comprising a portion of the homo sapiens protein tyrosine phosphatase, receptor-type, Z polypeptide (PTPRZ1) gene (NM_001206838) up to and including exon 1 (amino acid 19) and a portion of the MET gene (NM_000245) starting at exon 2 (amino acid 1). The slash after nucleotide 58 indicates the breakpoint (fusion junction) where translocation and in-frame fusion has occurred. The underlined codons at nucleotides 55-57 and 73-75 encode the last amino acid of PTPRZ1 and the first amino acid of MET, respectively. The shading at nucleotides 59-72 indicates 14 nucleotides from MET 5' UTR that are included in the fusion transcript.

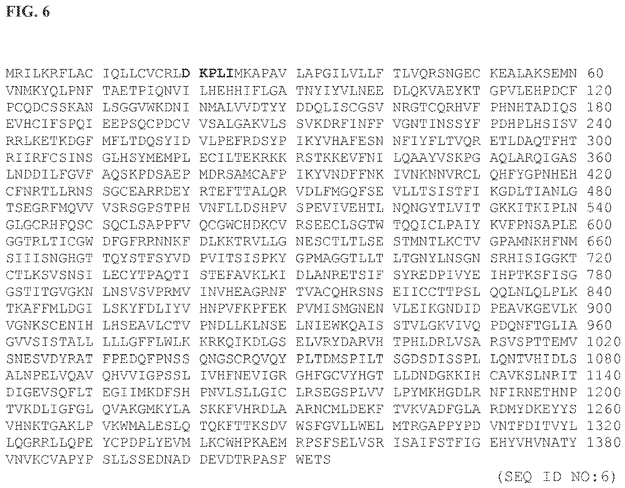

**FIG. 6** depicts the amino acid sequence of a PTPRZ1:MET fusion protein (SEQ ID NO:6). The amino acids in bold at positions 20-24 correspond to nucleotides 59-72 of SEQ ID NO:5. Because transcription starts in PTPRZ1, which is fused to a portion of the MET 5' non-coding sequence, the non-coding sequence is transcribed. The 5 amino acids from the MET 5' UTR are technically not part of the amino acid sequence for either PTPRZ1 or MET proteins.

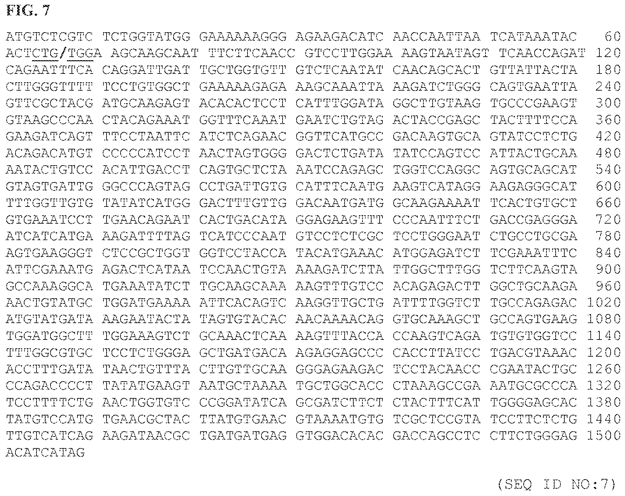

**FIG. 7** depicts the nucleotide sequence of an OXR1:MET gene fusion (SEQ ID NO:7) comprising a portion of the homo sapiens oxidation resistance 1 (OXR1) gene (NM_001198534) up to and including exon 1 (amino acid 22) and a portion of the MET gene (NM_000245) starting at exon 13 (amino acid 911). The underlined codons at nucleotides 64-66 and 67-69 encode the last amino acid of OXR1 and the first amino acid of MET, respectively. The slash between nucleotide 66 and 67 indicates the breakpoint or fusion junction where translocation and in-frame fusion has occurred.

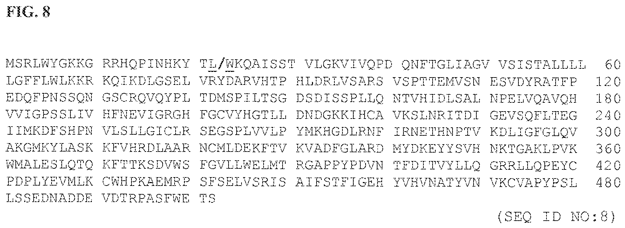

**FIG. 8** depicts the amino acid sequence of an OXR1:MET fusion protein (SEQ ID NO:8). The slash between amino acids 22 and 23 represents the location where the two proteins are fused and corresponds to nucleotides 64-66 and 67-69 of SEQ ID NO:7

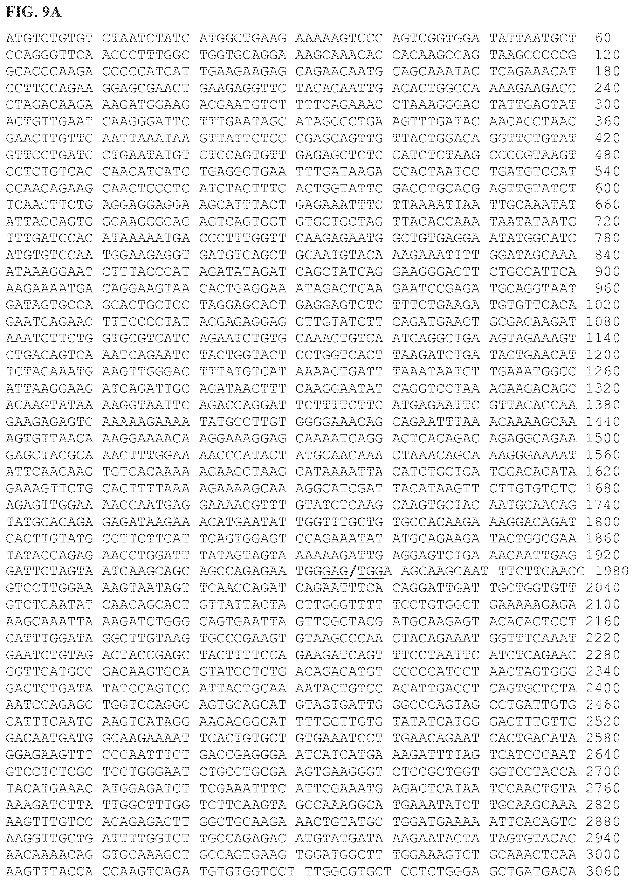

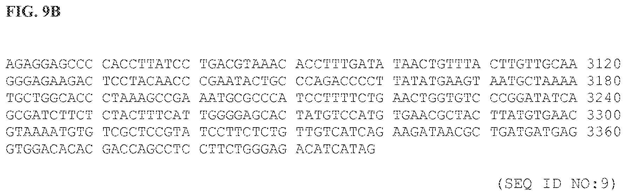

**FIG. 9A** **&** **9B** depict the nucleotide sequence of an OXR1:MET gene fusion (SEQ ID NO:9) comprising a portion of the OXR1gene (NM_018002) up to and including exon 11 (amino acid 652) and a portion of the MET gene (NM_000245) starting at exon 13 (amino acid 911). The underlined codons at nucleotides 1954-1956 and 1957-1959 encode the last amino acid of OXR1and the first amino acid of MET, respectively. The slash between nucleotide 1956 and 1957 indicates the breakpoint or fusion junction where translocation and in-frame fusion has occurred.

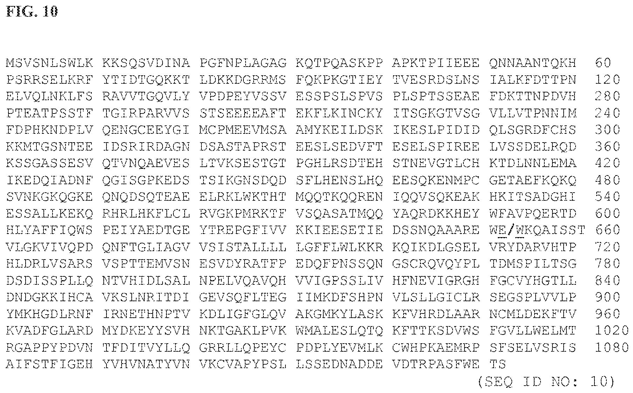

**FIG. 10** depicts the amino acid sequence of an OXR1:MET fusion protein (SEQ ID NO:10). The slash between amino acids 652 and 653 represents the location where the two proteins are fused and corresponds to nucleotides 1954-1956 and 1957-1959 of SEQ ID NO:9.

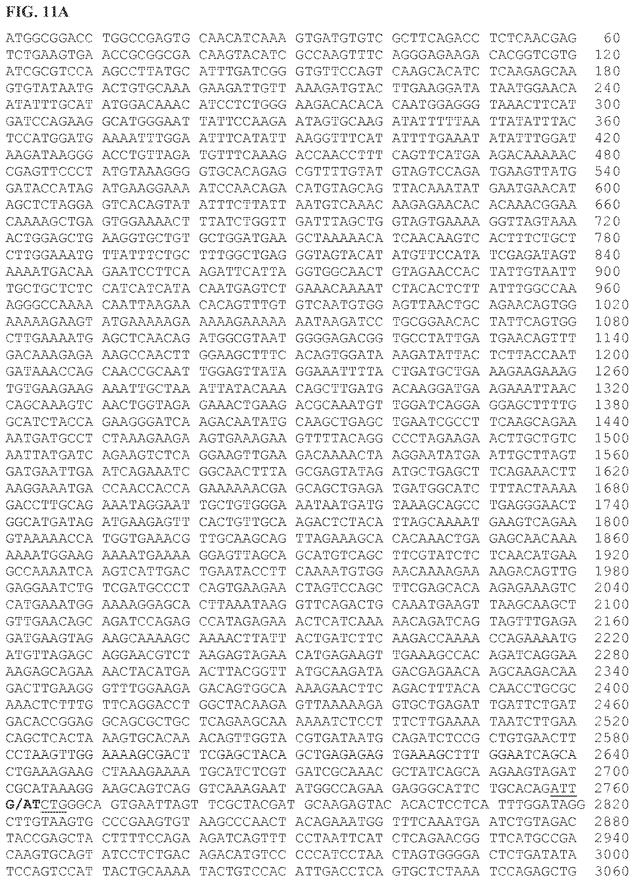

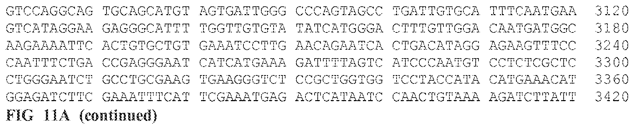

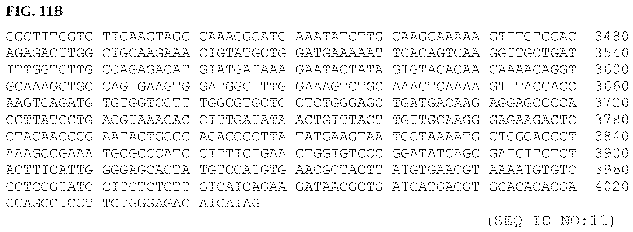

**FIG. 11A** **&** **11B** depict the nucleotide sequence of a KIF5B:MET gene fusion (SEQ ID NO:11) comprising a portion of the homo sapiens kinesin family member 5B (KIF5B) gene (NM_004521) up to and including exon 24 (amino acid 920) and a portion of the MET gene from exon 14 (amino acid 963). The underlined codons at nucleotides 2758-2760 and 2764-2766 encode the last amino acid of KIF5B and the first amino acid of MET, respectively. The slash between nucleotide 2761 and 2762 indicates the breakpoint (fusion junction) where translocation and in-frame fusion has occurred. The shading at nucleotides 2761-2763 indicates that nucleotides from both KIF5B and MET are fused in frame to form a codon and encode an amino acid.

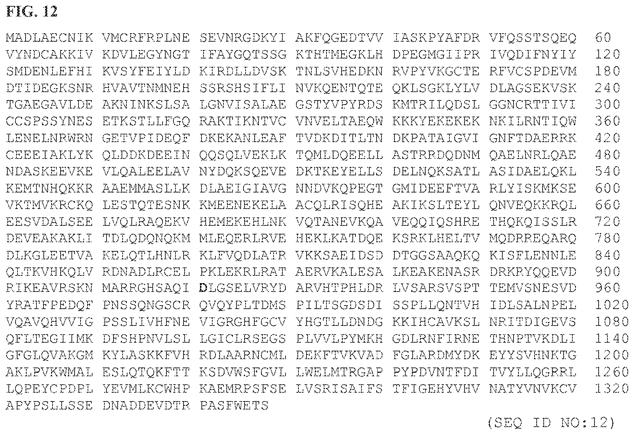

FIG. 12 depicts the amino acid sequence of a KIF5B:MET fusion protein (SEQ ID NO:12). The amino acid in bold at position 921 corresponds to nucleotides 2761-2763 of SEQ ID NO:11. This amino acid is encoded by nucleotides from both KIF5B and MET.

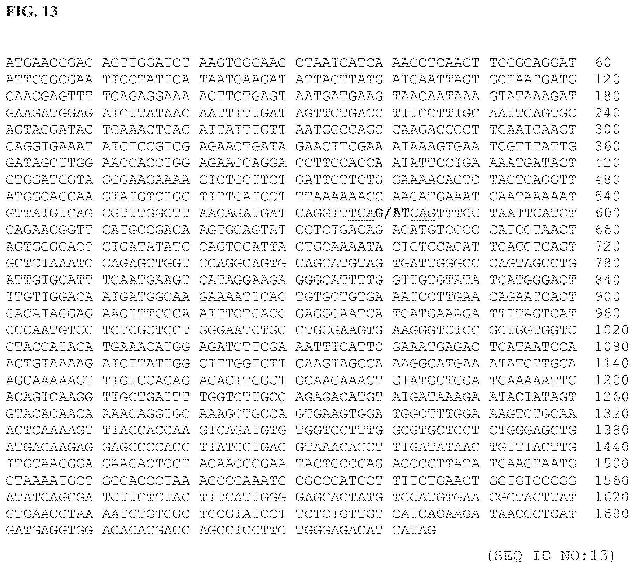

FIG. 13 depicts the nucleotide sequence of a TFG:MET gene fusion (SEQ ID NO:13) comprising a portion of the TRK-fused gene protein (TFG) gene up to and including exon 5 (amino acid 193) and a portion of the MET gene from exon number 15 (amino acid 1010). The underlined codons at nucleotides 577-579 and 583-585 encode the last amino acid of TFG and the first amino acid of MET, respectively. The slash after nucleotide 580 and 581 indicates the breakpoint (fusion junction) where translocation and in-frame fusion has occurred. The shading at nucleotides 580-582 indicates that nucleotides from both TFG and MET are fused in frame to form a codon and encode an amino acid.

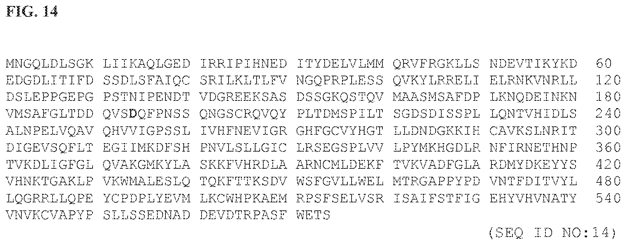

**FIG. 14** depicts the amino acid sequence of a TFG:MET fusion protein (SEQ ID NO:14). The amino acid in bold at position 194 corresponds to nucleotides 580-582 of SEQ ID NO:13. This amino acid is encoded by nucleotides from both TFG and MET.

### EXEMPLARY EMBODIMENTS OF THE INVENTION

The invention is based, at least in part, on the discovery of novel recombination or translocation events in cancer patients that result in at least a fragment of a MET gene linked to a non-homologous promoter via a recombination or translocation event that may result in aberrant expression and/or constitutive activation of MET kinase activity. Thus, a new patient population is identified, which is characterized by the presence of a MET fusion, e.g., a MET gene fusion or fusion protein. This new patient population suffers from or is susceptible to disorders mediated by aberrant MET expression or activity, or overexpression of MET, such as, e.g., a cancer. In another aspect, a new subtype of cancer is identified, which is characterized by the presence of the MET fusions described herein. In some embodiments, the new patient population suffers from or is susceptible to carcinoma of the kidney, brain, liver, lung, or thyroid characterized by the presence of a MET fusion. New methods of diagnosing and treating the patient population and the MET fusion cancer subtype are also provided.

MET is a membrane receptor that is involved in embryonic development and wound healing. It is a proto-oncogene that encodes a receptor with tyrosine kinase activity also known as hepatocyte growth factor (HGF) receptor. MET is normally expressed in epithelial cells and HGF is expressed in mesenchymal cells. Upon HGF stimulation, MET induces several biological responses that collectively give rise to invasive growth. Aberrant activation of the HGF/MET pathway leads to a variety of cancers. MET amplifications and mutations are also associated with a poor prognosis because they can trigger tumor growth, and formation of new blood vessels that supply the tumor with nutrients, and metastasis. In some embodiments, the MET fusions disclosed herein are associated with carcinomas of the kidney, brain, liver, lung, and thyroid. In some embodiments, the carcinomas associated with a MET fusion include papillary renal cell carcinoma, brain lower grade glioma, hepatocellular carcinoma, lung adenocarcinoma, and thyroid carcinoma.

The term "MET fusion" is used generically herein, and includes any fusion molecule (e.g., gene, gene product (e.g., cDNA, mRNA, or protein), and variants thereof) that includes a fragment of MET (in the case of a nucleotide sequence, particularly the coding region for the kinase domain of MET), and a fragment from a second non-homologous gene (in the case of a nucleotide sequence, the promoter and/or the coding region of the non-homologous gene). A MET fusion protein generally includes the kinase domain of MET. In some embodiments, the MET fusion can be, e.g., a BAIAP2L1:MET fusion, a C8orf34:MET fusion, a PTPRZ1:MET fusion, an OXR1:MET fusion, a KIF5B:MET fusion, or a TFG:MET fusion.

### MET Gene Fusions and Fusion Proteins

MET gene fusions are generated by a fusion between at least a part of the MET gene and a part of another gene as a result of a translocation (including inversion) within a chromosome or between chromosomes. As a result of a translocation, the MET gene may be placed under the transcriptional control of the partner gene promoter, resulting in aberrant expression or activity of MET, or overexpression of MET. Alternatively or additionally, the partner gene may include a dimerization domain that causes MET to become constitutively activated. As used herein, the 5'-region is upstream of, and the 3'-region is downstream of, a fusion junction or breakpoint in one of the component genes. MET and the gene or protein that it is fused to is referred to as "fusion partners." Alternatively, they may be identified as a "MET gene fusion" or a "MET fusion protein" which are collectively termed "MET fusions." The MET fusions disclosed herein have a kinase activity. The phrase "having a kinase activity" as used in this application means having an activity as an enzyme phosphorylating the side chain of an amino acid, such as tyrosine. In some embodiments, the MET fusion may include MET untranslated sequences (5' UTR), which, when fused in-frame to the fusion partner, results in transcription of nucleotides that are not normally included in the coding sequence and introduce amino acids into the fusion protein that are not part of MET or the fusion partner. See, e.g., FIGs. 5 and 6.

In some exemplary embodiments, the fusion partner is all or a portion of BAIAP2L1 (Brain-specific angiogenesis inhibitor 1-associated protein 2-like protein 1). In other exemplary embodiments, the fusion partner is all or a portion of C8orf34 (chromosome 8 open reading frame 34). In other exemplary embodiments, the fusion partner is all or a portion of PTPRZ1 (protein tyrosine phosphatase, receptor-type, Z polypeptide 1). In some embodiments, the fusion partner is all or a portion of OXR1(Oxidation resistance protein 1). In some embodiments, the fusion partner is all or a portion of KIF5B (Kinesin-1 heavy chain). In some embodiments, the fusion partner is all or a portion of TFG (TRK-fused gene protein).

Reference to "all or a portion" or "all or part" of a MET gene fusion or SEQ ID NO:1, 3, 5, 7, 9, 11, or 13, means that the nucleotide sequence comprises the entire MET gene fusion nucleotide sequence or a fragment of that sequence that comprises the fusion junction or breakpoint between MET and its fusion partner (such as, e.g., BAIAP2L1, C8orf34, PTPRZ1, OXR1, KIF5B, or TFG). The fragment may comprise 7, 8, 9, 10, 12, 14, 16, 18, 20, 21, 22, 23, 24, 25, 26, 27, 28, 29, 30, 35, 40, 45, 50, 60, 70, 80, 90, 100, 120, 150, 175, 200, 250, 300, or more nucleotides spanning the fusion junction of the MET gene fusion. Reference to "all or a portion" or "all or part" of a MET fusion protein or SEQ ID NO:2, 4, 6, 8, 10, 12, or 14, means an amino acid sequence that comprises the entire MET fusion protein amino acid sequence or a fragment of that sequence that comprises the fusion junction or breakpoint between MET and its fusion partner (such as, e.g., BAIAP2L1, C8orf34, PTPRZ1, OXR1, KIF5B, or TFG). The fragment may comprise 8, 10, 12, 14, 15, 16, 18, 20, 21, 22, 23, 24, 25, 26, 27, 28, 29, 30, 35, 40, 45, 50, 75, or more amino acids spanning the fusion junction.

In one embodiment of the invention, a fusion includes an in-frame fusion of all or a portion of the BAIAP2L1 gene (e.g., a BAIAP2L1 promotor or a functional fragment thereof, and one or more exons encoding BAIAP2L1 or a fragment thereof) and an exon of the MET gene (e.g., one or more exons encoding a MET kinase domain or a functional fragment thereof). Such a fusion can be referred to as a BAIAP2L1:MET fusion. In one embodiment, the BAIAP2L1:MET fusion comprises sufficient MET sequence to drive expression of a fusion protein that has kinase activity, e.g., has elevated activity as compared with wild type MET in the same tissue or cell.

In a particular embodiment, the invention provides a BAIAP2L1:MET gene fusion comprising the nucleotide sequence depicted in Figure 1 (SEQ ID NO:1), or a fragment thereof that includes the fusion junction. SEQ ID NO:1 comprises BAIAP2L1 (NM_018842) up to exon 9 (amino acid number 318) fused to MET (NM_000245), beginning at exon 15 (amino acid number 1011). In some embodiments the BAIAP2L1:MET gene fusion comprises a nucleotide sequence that is at least 85%, at least 90%, at least 95%, at least 97%, at least 98%, or at least 99% identical to all or part of SEQ ID NO:1. In some embodiments, the BAIAP2L1:MET gene fusion encodes a protein having the sequence depicted in Figure 2 (SEQ ID NO:2) or a sequence that is at least 85%, at least 90%, at least 95%, at least 97%, at least 98%, or at least 99% identical to all or part of SEQ ID NO:2.

In one embodiment of the invention, a fusion includes an in-frame fusion of all or a portion of the C8orf34 gene (e.g., a C8orf34 promotor or a functional fragment thereof, and one or more exons encoding C8orf34 or a fragment thereof) and an exon of the MET gene (e.g., one or more exons encoding a MET kinase domain or a functional fragment thereof). Such a fusion can be referred to as a C8orf34:MET fusion. In one embodiment, the C8orf34:MET fusion comprises sufficient MET sequence to drive expression of a fusion protein that has kinase activity, e.g., has elevated activity as compared with wild type MET in the same tissue or cell.

In a particular embodiment, the invention provides a C8orf34:MET gene fusion comprising the nucleotide sequence depicted in Figure 3 (SEQ ID NO:3), or a fragment thereof that includes the fusion junction. SEQ ID NO:1 comprises C8orf34 (NM_052958) up to exon 2 (amino acid number 158) fused to MET (NM_000245), beginning at exon 15 (amino acid number 1011). In some embodiments the C8orf34:MET gene fusion comprises a nucleotide sequence that is at least 85%, at least 90%, at least 95%, at least 97%, at least 98%, or at least 99% identical to all or part of SEQ ID NO:3. In some embodiments, the C8orf34:MET gene fusion encodes a protein having the sequence depicted in Figure 4 (SEQ ID NO:4) or a sequence that is at least 85%, at least 90%, at least 95%, at least 97%, at least 98%, at least 99% identical to all or part of SEQ ID NO:4.

In one embodiment of the invention, a fusion includes an in-frame fusion of all or a portion of the PTPRZ1 gene (e.g., a PTPRZ1 promotor or a functional fragment thereof, and one or more exons encoding PTPRZ1 or a fragment thereof) and an exon of the MET gene (e.g., one or more exons encoding a MET kinase domain or a functional fragment thereof). Such a fusion can be referred to as a PTPRZ1:MET fusion. In some embodiments, the PTPRZ1:MET fusion comprises sufficient MET sequence to drive expression of a fusion protein that has kinase activity, e.g., has elevated activity as compared with wild type MET in the same tissue or cell.

In a particular embodiment, the PTPRZ1:MET fusion has the nucleotide sequence depicted in Figure 5 (SEQ ID NO:5), or a fragment thereof that includes the fusion junction. SEQ ID NO:5 comprises PTPRZ1 (NM_001206838) up to and including exon 1 (amino acid number 19) fused to MET (NM_000245), beginning at exon 2 (amino acid 1). In some embodiments the PTPRZ1:MET gene fusion comprises a nucleotide sequence that is at least 85%, at least 90%, at least 95%, at least 97%, at least 98%, or at least 99% identical to all or part of SEQ ID NO:5. In some embodiments, the PTPRZ1:MET fusion encodes a protein having the sequence depicted in Figure 6 (SEQ ID NO:6) or a sequence that is at least 85%, at least 90%, at least 95%, at least 97%, at least 98%, or at least 99% identical to all or part of SEQ ID NO:6.

In one embodiment of the invention, a fusion includes an in-frame fusion of all or a portion of the OXR1gene (e.g., an OXR1promotor or a functional fragment thereof, and one or more exons encoding OXR1or a fragment thereof) and an exon of the MET gene (e.g., one or more exons encoding a MET kinase domain or a functional fragment thereof). Such a fusion can be referred to as an OXR1:MET fusion. In some embodiments, the OXR1:MET fusion comprises sufficient MET sequence to drive expression of a fusion protein that has kinase activity, e.g., has elevated activity as compared with wild type MET in the same tissue or cell.

In a particular embodiment, the OXR1:MET fusion has the nucleotide sequence depicted in Figure 7 (SEQ ID NO:7), or a fragment thereof that includes the fusion junction. SEQ ID NO:7 comprises OXR1(NM_001198534) up to and including exon 1 (amino acid number 22) fused to MET (NM_000245), beginning at exon 13 (amino acid number 911). In some embodiments the OXR1:MET gene fusion comprises a nucleotide sequence that is at least 85%, at least 90%, at least 95%, at least 97%, at least 98%, or at least 99% identical to all or part of SEQ ID NO:7. In some embodiments, the OXR1:MET fusion encodes a protein having the sequence depicted in Figure 8 (SEQ ID NO:8) or a sequence that is at least 85%, at least 90%, at least 95%, at least 97%, at least 98%, or at least 99% identical to all or part of SEQ ID NO:8.

In another particular embodiment, the OXR1:MET fusion has the nucleotide sequence depicted in Figure 9 (SEQ ID NO:9), or a fragment thereof that includes the fusion junction. SEQ ID NO:9 comprises OXR1(NM_018002) up to exon 11 (amino acid number 652) fused to MET (NM_000245), beginning at exon 13 (amino acid number 911). In some embodiments, the OXR1:MET gene fusion comprises a nucleotide sequence that is at least 85%, at least 90%, at least 95%, at least 97%, at least 98%, or at least 99% identical to all or part of SEQ ID NO:2. In some embodiments, the OXR1:MET fusion encodes a protein having the sequence depicted in Figure 10 (SEQ ID NO:10) or a sequence that is at least 85%, at least 90%, at least 95%, at least 97%, at least 98%, or at least 99% identical to all or part of SEQ ID NO:10.

In one embodiment of the invention, a fusion includes an in-frame fusion of all or a portion of the KIF5B gene (e.g., a KIF5B promotor or a functional fragment thereof, and one or more exons encoding KIF5B or a fragment thereof) and an exon of the MET gene (e.g., one or more exons encoding a MET kinase domain or a functional fragment thereof). Such a fusion can be referred to as a KIF5B:MET fusion. In one embodiment, the KIF5B:MET fusion comprises sufficient MET sequence to drive expression of a fusion protein that has kinase activity, e.g., has elevated activity as compared with wild type MET in the same tissue or cell.

In a particular embodiment, the invention provides a KIF5B:MET gene fusion comprising the nucleotide sequence depicted in Figure 11 (SEQ ID NO:11), or a fragment thereof that includes the fusion junction. SEQ ID NO:11 comprises KIF5B (NM_004521) up to and including exon 24 (amino acid number 920) fused to MET (NM_000245), beginning at exon 14 (amino acid number 963). In some embodiments the KIF5B:MET gene fusion comprises a nucleotide sequence that is at least 85%, at least 90%, at least 95%, at least 97%, at least 98%, or at least 99% identical to all or part of SEQ ID NO:11. In some embodiments, the KIF5B:MET gene fusion encodes a protein having the sequence depicted in Figure 12 (SEQ ID NO:12) or a sequence that is at least 85%, at least 90%, at least 95%, at least 97%, at least 98%, or at least 99% identical to all or part of SEQ ID NO:12.

In one embodiment of the invention, a fusion includes an in-frame fusion of all or a portion of the TFG gene (e.g., a TFG promotor or a functional fragment thereof, and one or more exons encoding a TFG or a fragment thereof) and an exon of the MET gene (e.g., one or more exons encoding a MET kinase domain or a functional fragment thereof). Such a fusion can be referred to as a TFG:MET fusion. In one embodiment, the TFG:MET fusion comprises sufficient MET sequence to drive expression of a fusion protein that has kinase activity, e.g., has elevated activity as compared with wild type MET in the same tissue or cell.

In a particular embodiment, the invention provides a TFG:MET gene fusion comprising the nucleotide sequence depicted in Figure 13 (SEQ ID NO:13), or a fragment thereof that includes the fusion junction. SEQ ID NO:13 comprises TFG (NM_0101007565) up to and including exon 5 (amino acid number 193) fused to MET (NM_000245), beginning at exon 15 (amino acid number 1010). In some embodiments the BAIAP2L1:MET gene fusion comprises a nucleotide sequence that is at least 85%, at least 90%, at least 95%, at least 97%, at least 98%, or at least 99% identical to all or part of SEQ ID NO:13. In some embodiments, the TFG:MET gene fusion encodes a protein having the sequence depicted in Figure 14 (SEQ ID NO:14) or a sequence that is at least 85%, at least 90%, at least 95%, at least 97%, at least 98%, or at least 99% identical to all or part of SEQ ID NO:14.

The nucleic acid sequences of MET gene fusions may be used as probes, primers, or bait to identify nucleotides from a biological sample that include, flank, or hybridize to MET fusions, such as, e.g., BAIAP2L1:MET (e.g., all or part of SEQ ID NO: 1), C8orf34:MET (e.g., all or part of SEQ ID NO:3), PTPRZ1:MET (e.g., all or part of SEQ ID NO:5), OXR1:MET (e.g., all or part of SEQ ID NO:7 or SEQ ID NO:9), KIF5B (e.g., all or part of SEQ ID NO:11), and TFG:MET (e.g., all or part of SEQ ID NO:13) at e.g., the fusion junctions. In certain embodiments, the probe, primer, or bait molecule is an oligonucleotide that allows capture, detection, and/or isolation of a MET gene fusion in a biological sample. In certain embodiments, the probes or primers derived from the nucleic acid sequences of MET gene fusions (e.g., from the fusion junctions) may be used, for example, for polymerase chain reaction (PCR) amplification. The oligonucleotide can comprise a nucleotide sequence substantially complementary to a fragment of the MET gene fusion nucleic acid molecules described herein. The sequence identity between the nucleic acid fragment, e.g., the oligonucleotide and the target MET gene fusion sequence, need not be exact, so long as the sequences are sufficiently complementary to allow the capture, detection, and/or isolation of the target sequence. In one embodiment, the nucleic acid fragment is a probe or primer that includes an oligonucleotide between about 5 and 25, e.g., between 10 and 20, or 10 and 15 nucleotides in length that includes the fusion junction of a MET fusion, such as, e.g., BAIAP2L1:MET (e.g., all or part of SEQ ID NO: 1), C8orf34:MET (e.g., all or part of SEQ ID NO:3), PTPRZ1:MET (e.g., all or part of SEQ ID NO:5), OXR1:MET (e.g., all or part of SEQ ID NO:7 or SEQ ID NO:9), KIF5B:MET (e.g., all or part of SEQ ID NO:11), and TFG:MET (e.g., all or part of SEQ ID NO:13). In other embodiments, the nucleic acid fragment is a bait that includes an oligonucleotide between about 100 to 300 nucleotides, 130 and 230 nucleotides, or 150 and 200 nucleotides in length that includes the fusion junction of a MET fusion, such as, e.g., BAIAP2L1:MET (e.g., all or part of SEQ ID NO: 1), C8orf34:MET (e.g., all or part of SEQ ID NO:3), PTPRZ1:MET (e.g., all or part of SEQ ID NO:5), OXR1:MET (e.g., all or part of SEQ ID NO:7 or SEQ ID NO:9), KIF5B:MET (e.g., all or part of SEQ ID NO:11), and TFG:MET (e.g., all or part of SEQ ID NO:13).

In certain embodiments, the nucleic acid fragments hybridize to a nucleotide sequence that includes a breakpoint or fusion junction, e.g., a breakpoint or fusion junction as identified by a slash ("/") in FIGs. 1, 3, 5, 7, 9, 11, and 13. For example, the nucleic acid fragment can hybridize to a nucleotide sequence that includes the fusion junction between the BAIAP2L1 transcript and the MET transcript (e.g., nucleotides 955-957 of SEQ ID NO:1), between the PTPRZ1 transcript and the MET transcript (e.g., nucleotides 58-60 of SEQ ID NO:5), between the C8orf34 transcript and the MET transcript (e.g., nucleotides 475-477 of SEQ ID NO:3), between the OXR1 transcript and the MET transcript (e.g., nucleotides 66-68 or 64-69 of SEQ ID NO:7 or nucleotides 1956-1958 or 1954-1959 of SEQ ID NO:9), between the KIF5B transcript and the MET transcript (e.g., nucleotides 2761-2763 of SEQ ID NO:11), or between the TFG transcript and the MET transcript (e.g., nucleotides 580-582 of SEQ ID NO:13), i.e., a nucleotide sequence that includes a portion of SEQ ID NO: 1, 3, 5, 7, 9, 11, or 13. Examples include a nucleotide sequence within exons 1-9 of a BAIAP2L1 gene and exons 15-21 of a MET gene (e.g., a portion of SEQ ID NO:1 comprising nucleotides 945-965, 930-980, or 905-1005 or nucleotides 954-958, 951-960, 946-965, 931-980, 906-1005, 881-1030, or 856-1055); a nucleotide sequence within exons 1 and 2 of a C8orf34 gene and exons 15-21 of a MET gene (e.g., a portion of SEQ ID NO:3 comprising nucleotides 474-478, 471-480, 466-485, 451-500, 426-525, 401-550, or 376-575); a nucleotide sequence within exon 1 of a PTPRZ1 gene and exons 2 to 21 of a MET gene (e.g., a portion of SEQ ID NO:5 comprising nucleotides 46-65, 31-80, or 6-105 or nucleotides 57-61, 54-63, 51-60, 45-64, 49-68, 34-83, 9-108, 2-151, or 2-201); a nucleotide sequence within exon 1 or within exon 1-11 of an OXR1 gene and exons 13-21 of a MET gene (e.g., the portion of SEQ ID NO:7 comprising nucleotides 56-75, 41-90, or 16-115 or nucleotides 65-69, 62-71, 57-76, 42-91, 17-116, 2-151, or 2-201, or the portion of SEQ ID NO:9 comprising nucleotides 1945-1964, 1931-1980, or 1905-2004 or nucleotides 1955-1959, 1952-1961, 1946-1965, 1931-1980, 1906-2005, 1881-2030, or 1856-2055); a nucleotide sequence within exon 1-24 of a KIF5B gene and exons 14-21 of a MET gene (e.g., the portion of SEQ ID NO:11 comprising nucleotides 2760-2764, 2757-2766, 2752-2771, 2737-2786, 2712-2811, 2687-2836, or 2662-2861); and a nucleotide sequence within exons 1-5 of a TFG gene and exons 15-21 of a MET gene (e.g., the portion of SEQ ID NO:13 comprising nucleotides 579-583, 576-585, 570-589 571-590, 556-605, 531-630, 506-655, or 481-680).

In other embodiments, the nucleic acid fragment includes a bait that comprises a nucleotide sequence that hybridizes to a MET gene fusion nucleic acid molecule described herein, and thereby allows the detection, capture, and/or isolation of the nucleic acid molecule. In one embodiment, a bait is suitable for solution phase hybridization. In other embodiments, a bait includes a binding entity or detection entity, e.g., an affinity tag or fluorescent label, that allows detection, capture, and/or separation, e.g., by binding to a binding entity, of a hybrid formed by a bait and a nucleic acid hybridized to the bait.

In exemplary embodiments, the nucleic acid fragments used as bait that includes a fusion junction between the BAIAP2L1 transcript and the MET transcript, e.g., a nucleotide sequence within SEQ ID NO:1 comprising nucleotides 955-957 (such as, e.g., a sequence comprising nucleotides 945-965, 930-980, or 905-1005 of SEQ ID NO:1 or nucleotides 954-958, 951-960, 946-965, 931-980, 906-1005, 881-1030, or 856-1055 of SEQ ID NO:1).

In other exemplary embodiments, the nucleic acid sequences hybridize to a nucleotide sequence that includes a fusion junction between the C8orf34 transcript and the MET transcript, e.g., a nucleotide sequence within SEQ ID NO:3 comprising nucleotides 475-477 (such as, e.g., a sequence comprising nucleotides 474-478, 471-480, 466-485, 451-500, 426-525, 401-550, or 376-575 of SEQ ID NO:3)

In other exemplary embodiments, the nucleic acid sequences hybridize to a nucleotide sequence that includes a fusion junction between the PTPRZ1 transcript and the MET transcript, e.g., a nucleotide sequence within SEQ ID NO:5 comprising nucleotides 58-60 (such as, e.g., a sequence comprising nucleotides 46-65, 31-80, or 6-105 of SEQ ID NO:5 or nucleotides 57-61, 54-63, 51-60, 45-64, 49-68, 34-83, 9-108, 2-151, or 2-201 of SEQ ID NO:5).

In other exemplary embodiments, the nucleic acid sequences hybridize to a nucleotide sequences that includes a fusion junction between the OXR1 transcript and the MET transcript, e.g., a nucleotide sequence within SEQ ID NO:7 comprising nucleotides 66-68 or 64-69 (such as, e.g., a sequence comprising nucleotides 56-75, 41-90, or 16-115 of SEQ ID NO:7 or 65-69, 62-71, 57-76, 42-91, 17-116, 2-151, or 2-201 of SEQ ID NO:7) or a nucleotide sequence within SEQ ID NO:9 comprising nucleotides 1956-1958 or 1954-1959 (such as, e.g., a sequence comprising nucleotides nucleotides 1945-1964, 1931-1980, or 1905-2004 of SEQ ID NO:9 or nucleotides 1955-1959, 1952-1961, 1946-1965, 1931-1980, 1906-2005, 1881-2030, or 1856-2055 of SEQ ID NO:9)

In other exemplary embodiments, the nucleic acid sequences hybridize to a nucleotide sequences that includes a fusion junction between the KIF5B transcript and the MET transcript, e.g., a nucleotide sequence within SEQ ID NO:11 comprising nucleotides 2761-2763 (such as, e.g., a sequence comprising nucleotides 2760-2764, 2757-2766, 2752-2771, 2737-2786, 2712-2811, 2687-2836, or 2662-2861 of SEQ ID NO:11).

In other exemplary embodiments, the nucleic acid sequences hybridize to a nucleotide sequences that includes a fusion junction between the TFG transcript and the MET transcript, e.g., a nucleotide sequence within SEQ ID NO:13 comprising nucleotides 580-582 (such as, e.g., a sequence comprising nucleotides 579-583, 576-585, 571-590, 570-589, 556-605, 531-630, 506-655, or 481-680 of SEQ ID NO:13).

Also disclosed herein are MET fusion proteins (such as, e.g., a purified or isolated BAIAP2L1:MET, PTPRZ1:MET, OXR1:MET, KIF5B:MET, or TFG:MET fusion protein), biologically active or antigenic fragments thereof, and use of those polypeptides for detecting and/or modulating the biological activity (such as tumorigenic activity) of a MET fusion protein. Exemplary MET fusion proteins comprise the amino acid sequence set forth in SEQ ID NO:2, 4, 6, 8, 10, 12, or 14, and fragments of those sequences.

### Detection and Diagnostic Methods

In another aspect, the invention provides a method of determining the presence of a MET gene fusion, such as, e.g., a BAIAP2L1:MET, a C8orf34:MET, a PTPRZ1:MET, an OXR1:MET, a KIF5B:MET, or a TFG:MET fusion as described herein. The presence of a MET gene fusion can indicate that the mammal providing the biological sample suffers from or is at risk of developing a disorder mediated by aberrant MET expression or activity, or overexpression of MET, such as, e.g., a cancer. The presence of a MET gene fusion may also indicate that the cancer is treatable with a MET inhibitor (such as, e.g., a kinase inhibitor or an antibody specific to MET) or a MET fusion inhibitor. In some embodiments, the cancer is carcinoma of the kidney, brain, liver, lung, or thyroid. In some embodiments, the cancer is selected from papillary renal cell carcinoma, brain lower grade glioma, hepatocellular carcinoma, lung adenocarcinoma, and thyroid carcinoma. In some embodiments, the MET fusion present in the sample is BAIAP2L1:MET and the cancer to be treated is renal papillary cell carcinoma. In other embodiments, the MET fusion present in the sample is C8orf34:MET and the cancer to be treated is renal papillary cell carcinoma. In certain embodiments, the MET fusion present in the sample is PTPRZ1:MET and the cancer to be treated is brain lower grade glioma. In other embodiments, the MET fusion present in the sample is OXR1:MET and the cancer to be treated is liver hepatocellular carcinoma. In some embodiments, the MET fusion present in the sample is KIF5B:MET and the cancer to be treated is lung adenocarcinoma. In other embodiments, the MET fusion present in the sample is TFG:MET and the cancer to be treated is thyroid carcinoma. In other embodiments, the cancer is a different cancer associated with aberrant expression or activity of MET or overexpression of MET.

In one embodiment, the MET fusion detected is a nucleic acid molecule or a polypeptide. The method includes detecting whether a MET fusion nucleic acid molecule or polypeptide is present in a cell (e.g., a circulating cell or a cancer cell), a tissue (e.g., a tumor), or a sample, e.g., a tumor sample, from a subject. In one embodiment, the sample is a nucleic acid sample. In one embodiment, the nucleic acid sample comprises DNA, e.g., genomic DNA or cDNA, or RNA, e.g., mRNA. In other embodiments, the sample is a protein sample.

The sample can be chosen from one or more sample types, such as, for example, tissue, e.g., cancerous tissue (e.g., a tissue biopsy), whole blood, serum, plasma, buccal scrape, sputum, saliva, cerebrospinal fluid, urine, stool, circulating tumor cells, circulating nucleic acids, or bone marrow.

### I. Methods for Detecting Gene Fusions

In some embodiments, the MET fusion is detected in a nucleic acid molecule by one or more methods chosen from nucleic acid hybridization assays (e.g. in situ hybridization, comparative genomic hybridization, microarray, Southern blot, northern blot), amplification-based assays (e.g., PCR, PCR-RFLP assay, or real-time PCR), sequencing and genotyping (e.g. sequence-specific primers, high-performance liquid chromatography, or mass-spectrometric genotyping), and screening analysis (including metaphase cytogenetic analysis by karyotype methods).

### (1) Hybridization methods

In some embodiments, the reagent hybridizes to a MET gene fusion, such as, e.g., nucleotides 945-965, 930-980, or 905-1005 of SEQ ID NO:1 or nucleotides 955-957, 954-958, 951-960, 946-965, 931-980, 906-1005, 881-1030, or 856-1055 of SEQ ID NO:1. In alternate embodiments, the reagent detects the presence of nucleotides 475-477, 474-478, 471-480, 466-485, 451-500, 426-525, 401-550, or 376-575 of SEQ ID NO:3, nucleotides 46-65, 31-80, or 6-105 of SEQ ID NO:5 or nucleotides 58-60, 57-61, 54-63, 51-60, 45-64, 49-68, 34-83, 9-108, 2-151, or 2-201 of SEQ ID NO:5, nucleotides 64-69, 56-75, 41-90, or 16-115 of SEQ ID NO:7 or nucleotides 66-68, 65-69, 62-71, 57-76, 42-91, 17-116, 2-151, or 2-201 of SEQ ID NO:7, nucleotides 1954-1959, 1945-1964, 1931-1980, or 1905-2004 of SEQ ID NO:9 or nucleotides 1956-1958, 1955-1959, 1952-1961, 1946-1965, 1931-1980, 1906-2005, 1881-2030, or 1856-2055 of SEQ ID NO:9, nucleotides 2761-2763, 2760-2764, 2757-2766, 2752-2771, 2737-2786, 2712-2811, 2687-2836, or 2662-2861 of SEQ ID NO:11, or nucleotides 580-582, 579-583, 576-585, 571-590, 570-589, 556-605, 531-630, 506-655, or 481-680 of SEQ ID NO:13. In an alternate embodiment, the method includes the steps of obtaining a sample; exposing the sample to a nucleic acid probe which hybridizes to an mRNA or cDNA encoding a MET fusion protein that comprises amino acids 317-321, 314-323, 309-328, or 294-344 of SEQ ID NO:2, amino acids 157-161, 155-164, 149-168, or 135-184 of SEQ ID NO:4, amino acids 20-24, 16-30, 10-34, or 2-51 of SEQ ID NO:6, amino acids 20-24, 17-26, 12-31, or 2-51 of SEQ ID NO:8, amino acids 650-604, 648-657, 642-661, 627-676 of SEQ ID NO:10, amino acids 919-923, 916-925, 911-930, or 896-945 of SEQ ID NO:12, or amino acids 192-196, 190-199, 184-203, or 170-219 of SEQ ID NO:14.

Hybridization, as described throughout the specification, may be carried out under stringent conditions, e.g., medium or high stringency. See, e.g., J. Sambrook, E.F. Fritsch, and T. Maniatis, Molecular Cloning: A Laboratory Manual, Cold Spring Harbor Laboratory Pr; 2nd edition (1989); T. Brown, Hybridization Analysis of DNA Blots. Current Protocols in Molecular Biology at 21:2.10.1-2.10.16 (2001). High stringency conditions for hybridization refer to conditions under which two nucleic acids must possess a high degree of base pair homology to each other in order to hybridize. Examples of highly stringent conditions for hybridization include hybridization in 4×sodium chloride/sodium citrate (SSC), at 65 or 70° C., or hybridization in 4×SSC plus 50% formamide at about 42 or 50° C., followed by at least one, at least two, or at least three washes in 1×SSC, at 65 or 70° C. Another example of highly stringent conditions includes hybridization in 2×SSC; 10×Denhardt solution (Fikoll 400+PEG+BSA;ratio 1:1:1); 0.1% SDS; 5 mM EDTA; 50 mM Na₂HPO₄;250 µg/ml of herring sperm DNA; 50 µg/ml of tRNA; or0.25 M of sodium phosphate buffer, pH 7.2;1 mM EDTA7% SDS at 60° C; followed by washing 2×SSC, 0.1% SDS at 60° C.

The nucleic acid fragments can be detectably labeled with, e.g., a radiolabel, a fluorescent label, a bioluminescent label, a chemiluminescent label, an enzyme label, a binding pair label (e.g., biotin/streptavidin), an antigen label, or can include an affinity tag, or identifier (e.g., an adaptor, barcode or other sequence identifier). Labeled or unlabeled nucleic acids and/or nucleic acid fragments may be used in reagents for detecting, capturing, and/or isolating MET gene fusions, such as, e.g., BAIAP2L1:MET (e.g., all or part of SEQ ID NO: 1), C8orf34:MET (e.g., all or part of SEQ ID NO:3), PTPRZ1:MET (e.g., all or part of SEQ ID NO:5), OXR1:MET (e.g., all or part of SEQ ID NO:7 or SEQ ID NO:9), KIF5B (e.g., all or part of SEQ ID NO:11), and TFG:MET (e.g., all or part of SEQ ID NO:13). In some embodiments, the labeled reagent can be detected using, e.g., autoradiography, microscopy (e.g., brightfield, fluorescence, or electron microscopy), enzyme-linked immunosorbent assay (ELISA), or immunohistochemistry.

In one embodiment, the method includes: contacting a nucleic acid sample, e.g., a genomic DNA sample (e.g., a chromosomal sample or a fractionated, enriched or otherwise pre-treated sample) or a gene product (mRNA, or cDNA), obtained from the subject, with a nucleic acid fragment e.g., a probe or primer as described herein (e.g., an exon-specific or a breakpoint-specific probe or primer) under conditions suitable for hybridization, and determining the presence or absence of the MET gene fusion, such as, e.g., BAIAP2L1:MET, C8orf34:MET, PTPRZ1:MET, OXR1:MET, KIF5B:MET, or TFG:MET as disclosed herein.

In some embodiments, the method comprises performing chromosome in situ hybridization with chromosomal DNA from a biological sample to detect the presence of a MET gene fusion (such as, e.g., BAIAP2L1:MET, C8orf34:MET, PTPRZ1:MET, OXR1:MET, KIF5B:MET, or TFG:MET as disclosed herein). In some embodiments, the chromosome in situ hybridization comprises the steps of: providing a chromosome (e.g., interphase or metaphase chromosome) preparation (e.g., by attaching the chromosomes to a substrate (e.g., glass)); denaturing the chromosomal DNA (e.g., by exposure to formamide) to separate the double strands of the polynucleotides from each other; exposing the nucleic acid probe to the chromosomes under conditions to allow hybridization of the probe to the target DNA; removing unhybridized or non-specifically hybridized probes by washing; and detecting the hybridization of the probe with the target DNA. In some embodiments, the chromosome in situ hybridization is fluorescence in situ hybridization (FISH). In some embodiments, the probe is labeled directly by a fluorescent label, or indirectly by incorporation of a nucleotide containing a tag or reporter molecule (e.g., biotin, digoxigenin, or hapten) which after hybridization to the target DNA is then bound by fluorescently labeled affinity molecule (e.g., an antibody or streptavidin). In some embodiments, the hybridization of the probe with the target DNA in FISH can be visualized using a fluorescence microscope.

In other embodiments, the method comprises performing Southern blot with DNA polynucleotides from a biological sample to detect the presence of a MET gene fusion (such as, e.g., BAIAP2L1:MET, C8orf34:MET, PTPRZ1:MET, OXR1:MET, KIF5B:MET, or TFG:MET as disclosed herein). In some embodiments, the Southern blot comprises the steps of: optionally fragmenting the polynucleotides into smaller sizes by restriction endonucleases; separating the polynucleotides by gel electrophoresis; denaturing the polynucleotides (e.g., by heat or alkali treatment) to separate the double strands of the polynucleotides from each other; transferring the polynucleotides from the gel to a membrane (e.g., a nylon or nitrocellulose membrane); immobilizing the polynucleotides to the membrane (e.g., by UV light or heat); exposing the nucleic acid probe to the polynucleotides under conditions to allow hybridization of the probe to the target DNA; removing unhybridized or non-specifically hybridized probes by washing; and detecting the hybridization of the probe with the target DNA.

### (2) Amplification-based assays

In certain embodiments, the method of determining the presence of a MET gene fusion, comprises (a) performing a PCR amplification reaction with polynucleotides from a biological sample, wherein the amplification reaction utilizes a pair of primers which will amplify at least a fragment of the MET gene fusion, wherein the fragment comprises the fusion junction, wherein the first primer is in sense orientation and the second primer is in antisense orientation; and (b) detecting an amplification product, wherein the presence of the amplification product is indicative of the presence of a MET fusion polynucleotide in the sample. In specific exemplary embodiments, the MET gene fusion is BAIAP2L1:MET, such as, e.g., the gene fusion of SEQ ID NO: 1 or a fragment thereof comprising nucleotides 955-957, 954-958, 951-960, 946-965, 931-980, 906-1005, 881-1030, or 856-1055 of SEQ ID NO:1 or nucleotides 946-965, 930-980, or 905-1005 of SEQ ID NO:1 In other exemplary embodiments, the gene fusion is C8orf34:MET such as, e.g. the gene fusion of SEQ ID NO:3 or a fragment thereof comprising nucleotides 475-477, 474-478, 471-480, 466-485, 451-500, 426-525, 401-550, or 376-575 of SEQ ID NO:3. In other exemplary embodiments, the gene fusion is PTPRZ1:MET such as, e.g. the gene fusion of SEQ ID NO:5 or a fragment thereof comprising nucleotides 58-60, 57-61, 54-63, 51-60, 45-64, 49-68, 34-83, 9-108, 2-151, or 2-201 of SEQ ID NO:5 or nucleotides 51-60, 46-65, 31-80, 6-105 of SEQ ID NO:5. In certain exemplary embodiments, the gene fusion is OXR1:MET such as, e.g. the gene fusion of SEQ ID NO:7 or a fragment thereof comprising nucleotides 66-68, 65-69, 62-71, 57-76, 42-91, 17-116, 2-151, or 2-201 of SEQ ID NO:7 or nucleotides 65-68, 64-69, 62-71, 56-75, 41-90, or 16-115 of SEQ ID NO:7 or the gene fusion of SEQ ID NO:9 or a fragment thereof comprising nucleotides 1954-1959, 1945-1964, 1931-1980, or 1905-2004 of SEQ ID NO:9 or nucleotides 1955-1958, 1955-1959, 1952-1961, 1946-1965, 1931-1980, 1906-2005, 1881-2030, or 1856-2055 of SEQ ID NO:9. In other exemplary embodiments, the gene fusion is KIF5B:MET such as, e.g. the gene fusion of SEQ ID NO:11 or a fragment thereof comprising nucleotides 2761-2763, 2760-2764, 2757-2766, 2752-2771, 2737-2786, 2712-2811, 2687-2836, or 2662-2861 of SEQ ID NO:11. In some exemplary embodiments, the gene fusion is TFG:MET such as, e.g. the gene fusion of SEQ ID NO:13 or a fragment thereof comprising nucleotides 580-582, 579-583, 576-585, 570-589, 571-590, 556-605, 531-630, 506-655, or 481-680 of SEQ ID NO:13.

In some embodiments, step (a) of performing a PCR amplification reaction comprises: (i) providing a reaction mixture comprising the polynucleotides (e.g., DNA or cDNA) from the biological sample, the pair of primers which will amplify at least a fragment of the MET gene fusion wherein the first primer is complementary to a sequence on the first strand of the polynucleotides and the second primer is complementary to a sequence on the second strand of the polynucleotides, a DNA polymerase, and a plurality of free nucleotides comprising adenine, thymine, cytosine, and guanine (dNTPs); (ii) heating the reaction mixture to a first predetermined temperature for a first predetermined time to separate the double strands of the polynucleotides from each other; (iii) cooling the reaction mixture to a second predetermined temperature for a second predetermined time under conditions to allow the first and second primers to hybridize with their complementary sequences on the first and second strands of the polynucleotides, and to allow the DNA polymerase to extend the primers; and (iv) repeating steps (ii) and (iii) for a predetermined number of cycles (e.g., 10, 15, 20, 25, 30, 35, 40, 45, or 50 cycles).

In some embodiments, the polynucleotides from the biological sample comprise RNA, and the method further comprises performing a RT-PCR amplification reaction with the RNA to synthesize cDNA as the template for subsequent or simultaneous PCR reactions. In some embodiments, the RT-PCR amplification reaction comprises providing a reaction mixture comprising the RNA, a primer which will amplify the RNA (e.g., a sequence-specific primer, a random primer, or oligo(dT)s), a reverse transcriptase, and dNTPs, and heating the reaction mixture to a third predetermined temperature for a third predetermined time under conditions to allow the reverse transcriptase to extend the primer.

### (3) Sequencing and Genotyping

Another method for determining the presence of a MET gene fusion molecule (such as, e.g., BAIAP2L1:MET, C8orf34:MET, PTPRZ1:MET, OXR1:MET, KIF5B:MET, or TFG:MET as disclosed herein) includes: sequencing a portion of the nucleic acid molecule (e.g., sequencing the portion of the nucleic acid molecule that comprises the fusion junction of a MET gene fusion), thereby determining that the MET gene fusion is present in the nucleic acid molecule. Optionally, the sequence acquired is compared to a reference sequence, or a wild type reference sequence. In one embodiment, the sequence is determined by a next generation sequencing method. In some embodiments, the sequencing is automated and/or highthroughput sequencing. The method can further include acquiring, e.g., directly or indirectly acquiring, a sample, e.g., a tumor or cancer sample, from a patient.

In some embodiments, the sequencing comprises chain terminator sequencing (Sanger sequencing), comprising: providing a reaction mixture comprising a nucleic acid molecule from a biological sample, a primer complementary to a region of the template nucleic acid molecule, a DNA polymerase, a plurality of free nucleotides comprising adenine, thymine, cytosine, and guanine (dNTPs), and at least one chain terminating nucleotide (e.g., at least one dideoxynucleotide (ddNTPs) chosen from ddATP, ddTTP, ddCTP, and ddGTP), wherein the at least one chain terminating nucleotide is present in a low concentration so that chain termination occurs randomly at any one of the positions containing the corresponding base on the DNA strand; annealing the primer to a single strand of the nucleic acid molecule; extending the primer to allow incorporation of the chain terminating nucleotide by the DNA polymerase to produce a series of DNA fragments that are terminated at positions where that particular nucleotide is used; separating the polynucleotides by electrophoresis (e.g., gel or capillary electrophoresis); and determining the nucleotide order of the template nucleic acid molecule based on the positions of chain termination on the DNA fragments. In some embodiments, the sequencing is carried out with four separate base-specific reactions, wherein the primer or the chain terminating nucleotide in each reaction is labeled with a separate fluorescent label. In other embodiments, the sequencing is carried out in a single reaction, wherein the four chain terminating nucleotides mixed in the single reaction are each labeled with a separate fluorescent label.

In some embodiments, the sequencing comprises pyrosequencing (sequencing by synthesis), comprising: (i) providing a reaction mixture comprising a nucleic acid molecule from a biological sample, a primer complementary to a region of the template nucleic acid molecule, a DNA polymerase, a first enzyme capable of converting pyrophosphate into ATP, and a second enzyme capable using ATP to generates a detectable signal (e.g., a chemiluminescent signal, such as light) in an amount that is proportional to the amount of ATP; (ii) annealing the primer to a single strand of the nucleic acid molecule; (iii) adding one of the four free nucleotides (dNTPs) to allow incorporation of the correct, complementary dNTP onto the template by the DNA polymerase and release of pyrophosphate stoichiometrically; (iv) converting the released pyrophosphate to ATP by the first enzyme; (v) generating a detectable signal by the second enzyme using the ATP; (vi) detecting the generated signal and analyzing the amount of signal generated in a pyrogram; (vii) removing the unincorporated nucleotides; and (viii) repeating steps (iii) to (vii). The method allows sequencing of a single strand of DNA, one base pair at a time, and detecting which base was actually added at each step. The solutions of each type of nucleotides are sequentially added and removed from the reaction. Light is produced only when the nucleotide solution complements the first unpaired base of the template. The order of solutions which produce detectable signals allows the determination of the sequence of the template.

In some embodiments, the method of determining the presence of a MET fusion (such as, e.g., BAIAP2L1:MET, C8orf34:MET, PTPRZ1:MET, OXR1:MET, KIF5B:MET, or TFG:MET as disclosed herein) comprises analyzing a nucleic acid sample (e.g., DNA, cDNA, or RNA, or an amplification product thereof) by HPLC. The method may comprise: passing a pressurized liquid solution containing the sample through a column filled with a sorbent, wherein the nucleic acid or protein components in the sample interact differently with the sorbent, causing different flow rates for the different components; separating the components as they flow out the column at different flow rates. In some embodiments, the HPLC is chosen from, e.g., reverse-phase HPLC, size exclusion HPLC, ion-exchange HPLC, and bioaffinity HPLC.

In some embodiments, the method of determining the presence of a MET fusion (such as, e.g., BAIAP2L1:MET, C8orf34:MET, PTPRZ1:MET, OXR1:MET, KIF5B:MET, or TFG:MET as disclosed herein) comprises analyzing a nucleic acid sample (e.g., DNA, cDNA, or RNA, or an amplification product thereof) by mass spectrometry. The method may comprise: ionizing the components in the sample (e.g., by chemical or electron ionization); accelerating and subjecting the ionized components to an electric or magnetic field; separating the ionized components based on their mass-to-charge ratios; and detecting the separated components by a detector capable of detecting charged particles (e.g., by an electron multiplier).

Detection of a MET gene fusion or a MET fusion protein in a patient can lead to assignment of the patient to the newly identified patient population that bears the MET fusion. Because this patient population can suffer from or be susceptible to a disorder associated with an aberrant MET expression or activity or overexpression of MET, detection of the MET fusion can also lead to diagnosis of such disorder. Thus, also disclosed herein is a method of stratifying a patient population (e.g., assigning a patient, to a group or class) and/or diagnosing a patient, comprising: obtaining a biological sample from the patient, contacting the sample with at least one reagent that detects a MET gene fusion or a MET fusion protein to determine whether a MET fusion is present in the biological sample. The detection of a MET fusion indicates that the patient belongs to the newly identified patient population that bears the MET fusion, and/or the presence of a disorder associated with aberrant MET expression or activity or overexpression or MET, such as e.g., a cancer. The detection of a MET fusion also identifies a new subtype of cancer, which is characterized by the presence of the MET fusion, such as e.g., carcinoma of the kidney, brain, liver, lung, or thyroid. In some embodiments, the cancer is selected from papillary renal cell carcinoma, brain lower grade glioma, hepatocellular carcinoma, lung adenocarcinoma, and thyroid carcinoma. In certain embodiments, the MET fusion is BAIAP2L1:MET. In other embodiments, the MET fusion is C8orf34:MET, PTPRZ1:MET, OXR1:MET, KIF5B:MET, or TFG:MET. In some embodiments, the BAIAP2L1:MET fusion has all or a part of the nucleotide and/or amino acid sequence (such as, e.g., the fusion junction) set forth in SEQ ID NO:1 and SEQ ID NO:2, respectively. In some embodiments, the C8orf34:MET fusion has all or part of the nucleotide and/or amino acid sequence (such as, e.g., the fusion junction) set forth in SEQ ID NO:3 and SEQ ID NO:4, respectively. In some embodiments, the PTPRZ1:MET fusion has all or part of the nucleotide and/or amino acid sequence (such as, e.g., the fusion junction) set forth in SEQ ID NO:5 and SEQ ID NO:6, respectively. In some embodiments, the OXR1:MET fusion has all or part of the nucleotide and/or amino acid sequence (such as, e.g., the fusion junction) set forth in SEQ ID NO:7 and SEQ ID NO:8, respectively. In some embodiments, the OXR1:MET fusion has all or part of the nucleotide and/or amino acid sequence (such as, e.g., the fusion junction) set forth in SEQ ID NO:9 and SEQ ID NO:10, respectively. In some embodiments, the KIF5B:MET fusion has all or part of the nucleotide and/or amino acid sequence (such as, e.g., the fusion junction) set forth in SEQ ID NO:11 and SEQ ID NO:12, respectively. In some embodiments, the TFG:MET fusion has all or part of the nucleotide and/or amino acid sequence (such as, e.g., the fusion junction) set forth in SEQ ID NO:13 and SEQ ID NO:14, respectively.

In certain embodiments, upon detection of a MET gene fusion in a patient's biological sample, the patient is identified as likely to respond to a treatment that comprises a MET inhibitor, or a MET fusion inhibitor. In some embodiments, the MET fusion detected is a BAIAP2L1:MET fusion. In alternate embodiments, the MET fusion detected is a PTPRZ1:MET fusion. In some embodiments, the MET fusion detected is an OXR1:MET fusion. In some embodiments the MET fusion detected is a C8orf34:MET fusion. In some embodiments, the MET fusion detected is a KIF5B:MET fusion. In certain embodiments, the MET fusion detected is a TFG:MET fusion.

### Methods for Validating MET Fusions

MET gene fusions, such as, e.g., MET gene fusions (e.g., BAIAP2L1:MET gene fusions, PTPRZ1:MET gene fusions, OXR1:MET gene fusions, C8orf34:MET gene fusions, KIF5B:MET gene fusions, or TFG:MET gene fusions) may be evaluated to ensure that the breakpoints are in-frame and can produce a protein product containing the full kinase domain, i.e., that the breakpoint occurs such that complete triplet codons are intact, and that the RNA sequence will produce a viable protein. The MET gene fusion can be transfected into cells to confirm that the protein is functionally active with respect to kinase activity and oncogenic activity. cDNA encoding the MET fusion protein can be produced by standard solidphase DNA synthesis. Alternatively the MET fusion cDNA can be produced by RT-PCR using tumor mRNA extracted from samples containing the gene fusion. The DNA amplified can be subcloned into an appropriate vector and characterized by DNA sequence analysis or in vitro/in vivo expression analyses.

Expression vectors containing the MET gene fusion (such as, e.g., a MET gene fusion, e.g., a BAIAP2L1:MET gene fusion, a PTPRZ1:MET gene fusion, an OXR1:MET gene fusion, a C8orf34:MET gene fusion, a KIF5B:MET gene fusion, or a TFG:MET gene fusion) can be introduced into host cells to thereby produce a MET fusion protein (such as, e.g., a MET fusion protein, e.g., a BAIAP2L1:MET fusion protein, a PTPRZ1:MET fusion protein, an OXR1:MET fusion protein, a C8orf34:MET fusion protein, a KIF5B:MET fusion protein, or a TFG:MET fusion protein). The MET fusion protein expression vector can be a yeast expression vector, a vector for expression in insect cells, e.g., a baculovirus expression vector, or a vector suitable for expression in mammalian cells. Vector DNA can be introduced into host cells via conventional transformation or transfection techniques. As used herein, the terms "transformation" and "transfection" are intended to refer to a variety of art-recognized techniques for introducing foreign nucleic acid (e.g., DNA) into a host cell.

Cells harboring the expression vector carrying the recombinant MET gene fusion can then be tested for production of the unique fusion protein via standard Western blotting using either an antibody probe that detects the gene product itself or that recognizes a tag peptide (e.g., FLAG tag) that can be added to the gene product via the expression vector (using standard, commercially available reagents). Western blotting can be used to confirm the ectopic expression of the encoded MET fusion protein by comparing the samples from cells transfected with the vector containing the MET gene fusion cDNA to cells transfected with the empty expression vector. The functional activity can be assessed by measuring the level of phosphorylation on the kinase or substrate. Comparison of the level of phosphorylation activity between the wild type (normal) form of MET and the MET fusion protein can indicate if the MET fusion protein has elevated activity that could drive oncogenic activity. Whether the MET gene fusion is oncogenic can be assessed by measuring capacity of the expressed MET fusion protein to transform cells, that is, to enable cells to grow and proliferate under conditions which are not permissive for growth of normal cells. One commonly used method of measuring the transforming activity of a kinase is by assessing if expression of the gene product can allow BaF3 cells to grow in the absence of the growth factor IL3, which is required for the survival and growth of BaF3 cells. Another assay for measuring transforming activity is a soft agar growth assay. This is another standard method which tests the capacity of an introduced gene product to confer the ability to grow in a soft agar matrix, or anchorage-independent conditions. These methods and others can be used to test the oncogenic activity of a MET gene fusion (such as, e.g., a BAIAP2L1:MET gene fusion, a PTPRZ1:MET gene fusion, a C8orf34:MET gene fusion, a KIF5B:MET gene fusion, or a TFG:MET gene fusion) and provide a level of validation of a MET fusion protein (such as, e.g., a BAIAP2L1:MET fusion protein, a PTPRZ1:MET fusion protein, an OXR1:MET fusion protein, a C8orf34:MET fusion protein, a KIF5B:MET fusion protein, or a TFG:MET fusion protein) as a potential target for treating patients that harbor these fusions.

A change in an activity of a cell can be detected in a cell in culture, e.g., a cell expressing a fusion (e.g., a mammalian cell, a tumor cell or cell line, a recombinant cell). The transfected cell can show a change in response to the expressed fusion, e.g., increased proliferation, changes in morphology, increased tumorigenicity, and/or an acquired transformed phenotype.

To further validate the biological implication of the gene fusion, a change in any of the activities of the cell, e.g., the recombinant cell, in the presence of a known inhibitor of one of the fusion partners, e.g., a MET inhibitor, can be detected. For example, a decrease in one or more of: proliferation, tumorigenicity, and transformed morphology, in the presence of the MET inhibitor can be indicative of an inhibitor of a fusion. In other embodiments, a change in binding activity or phosphorylation of MET or its interacting or downstream proteins or molecules is detected.

## Claims

1. A molecule capable of specifically binding to a BAIAP2L1:MET, C8orf34:MET, PTPRZ1:MET, OXR1:MET, KIF5B:MET, or TFG:MET gene fusion, wherein the molecule is an oligonucleotide that hybridizes to the fusion junction of a MET gene fusion selected from BAIAP2L1:MET, C8orf34:MET, PTPRZ1:MET, OXR1:MET, KIF5B:MET, and TFG:MET
wherein
i) the BAIAP2L1 :MET gene fusion to which the oligonucleotide binds comprises SEQ ID NO:1, or a portion of SEQ ID NO:1, wherein the portion comprises the fusion junction between BAIAP2L1 and MET,
ii) the C8orf34:MET gene fusion to which the oligonucleotide binds comprises SEQ ID NO:3, or a portion of SEQ ID NO:3, wherein the portion comprises the fusion junction between C8orf34 and MET,
iii) the PTPRZ1 :MET gene fusion to which the oligonucleotide binds comprises SEQ ID NO:5, or a portion of SEQ ID NO:5, wherein the portion comprises the fusion junction between PTPRZ1 and MET,
iv) the OXR1:MET gene fusion to which the oligonucleotide binds comprises SEQ ID NO:7 or 9, or a portion of SEQ ID NO:7 or 9, wherein the portion comprises the fusion junction between OXR1 and MET,
v) the KIF5B:MET gene fusion to which the oligonucleotide binds comprises SEQ ID NO:11, or a portion of SEQ ID NO:11, wherein the portion comprises the fusion junction between KIF5B and MET, or
vi) the TFG:MET gene fusion to which the oligonucleotide binds comprises SEQ ID NO:13, or a portion of SEQ ID NO:13, wherein the portion comprises the fusion junction between TFG and MET.

2. The molecule of claim 1, wherein the fusion results in aberrant activity or expression of MET or overexpression of MET.

3. The molecule of any one of claims 1 to 2, wherein the molecule is an oligonucleotide that specifically hybridizes to:
a) at least nucleotides 951-960 of SEQ ID NO:1;
b) at least nucleotides 471-480 of SEQ ID NO:3;
c) at least nucleotides 54-63 of SEQ ID NO:5;
d) at least nucleotides 62-71 of SEQ ID NO:7;
e) at least nucleotides 1952-1961 of SEQ ID NO:9;
f) at least nucleotides 2757-2766 of SEQ ID NO:11;
g) at least nucleotides 576-585 of SEQ ID NO:13; or
h) a complement of any one of a) - g).

4. A method of detecting a MET gene fusion in a biological sample, comprising contacting a biological sample from a mammal with a molecule according to any one of claims 1 to 3, wherein the detection of the MET gene fusion indicates the presence of a MET fusion in the biological sample.

5. A method for diagnosing cancer in a patient, the method comprising contacting a biological sample from the patient with a molecule according to any one of claims 1 to 3 to determine whether a MET gene fusion is present in the biological sample, wherein the detection of the MET gene fusion indicates the presence of or susceptibility to cancer.

6. The method according to claim 5, wherein the cancer is: carcinoma of the kidney; carcinoma of the brain; carcinoma of the liver; carcinoma of the lung; carcinoma of the thyroid; papillary renal cell carcinoma; brain lower grade glioma; hepatocellular carcinoma; lung adenocarcinoma; and/or thyroid carcinoma.

## Patentansprüche

1. Molekül, das in der Lage ist, spezifisch an eine Genfusion BAIAP2L1:MET, C8orf34:MET, PTPRZ1:MET, OXR1:MET, KIF5B:MET oder TFG:MET zu binden, wobei das Molekül ein Oligonukleotid ist, das an den Fusionsübergang einer MET-Genfusion, ausgewählt aus BAIAP2L1:MET, C8orf34:MET, PTPRZ1:MET, OXR1:MET, KIF5B:MET, und TFG:MET, hybridisiert
wobei
i) die Genfusion BAIAP2L1:MET, an die das Oligonukleotid bindet, SEQ ID NO: 1 oder einen Abschnitt von SEQ ID NO: 1 umfasst, wobei der Abschnitt den Fusionsübergang zwischen BAIAP2L1 und MET umfasst,
ii) die Genfusion C8orf34:MET, an die das Oligonukleotid bindet, SEQ ID NO: 3 oder einen Abschnitt von SEQ ID NO: 3 umfasst, wobei der Abschnitt den Fusionsübergang zwischen C8orf34 und MET umfasst,
iii) die Genfusion PTPRZ1:MET, an die das Oligonukleotid bindet, SEQ ID NO: 5 oder einen Abschnitt von SEQ ID NO: 5 umfasst, wobei der Abschnitt den Fusionsübergang zwischen PTPRZ1 und MET umfasst,
iv) die Genfusion OXR1:MET, an die das Oligonukleotid bindet, SEQ ID NO: 7 oder 9, oder einen Abschnitt von SEQ ID NO: 7 oder 9 umfasst, wobei der Abschnitt den Fusionsübergang zwischen OXR1 und MET umfasst,
v) die Genfusion KIF5B:MET, an die das Oligonukleotid bindet, SEQ ID NO: 11 oder einen Abschnitt von SEQ ID NO: 11 umfasst, wobei der Abschnitt den Fusionsübergang zwischen KIF5B und MET umfasst, oder
vi) die Genfusion TFG:MET, an die das Oligonukleotid bindet, SEQ ID NO: 13 oder einen Abschnitt von SEQ ID NO: 13 umfasst, wobei der Abschnitt den Fusionsübergang zwischen TFG und MET umfasst, oder

2. Molekül nach Anspruch 1, wobei die Fusion in einer aberranten Aktivität oder Expression von MET oder einer Überexpression von MET resultiert.

3. Molekül nach einem der Ansprüche 1 bis 2, wobei das Molekül ein Oligonukleotid ist, das spezifisch hybridisiert an:
a) mindestens die Nukleotide 951-960 von SEQ ID NO: 1;
b) mindestens die Nukleotide 471-480 von SEQ ID NO: 3;
c) mindestens die Nukleotide 54-63 von SEQ ID NO: 5;
d) mindestens die Nukleotide 62-71 von SEQ ID NO: 7;
e) mindestens die Nukleotide 1952-1961 von SEQ ID NO: 9;
f) mindestens die Nukleotide 2757-2766 von SEQ ID NO: 11;
g) mindestens die Nukleotide 576-585 von SEQ ID NO: 13; oder
h) ein Komplement von einem beliebigen von a) - g).

4. Verfahren zum Detektieren einer MET-Genfusion in einer biologischen Probe, umfassend ein Inkontaktbringen einer biologischen Probe von einem Säugetier mit einem Molekül nach einem der Ansprüche 1 bis 3, wobei die Detektion der MET-Genfusion indikativ für das Vorhandensein einer MET-Fusion in der biologischen Probe ist.

5. Verfahren zur Diagnose von Krebs bei einem Patienten, wobei das Verfahren ein Inkontaktbringen einer biologischen Probe des Patienten mit einem Molekül nach einem der Ansprüche 1 bis 3 umfasst, um zu bestimmen, ob eine MET-Genfusion in der biologischen Probe vorhanden ist, wobei die Detektion der MET-Genfusion indikativ für das Vorhandensein von Krebs oder die Anfälligkeit dafür ist.

6. Verfahren nach Anspruch 5, wobei der Krebs Folgender ist: Nierenkarzinom, Hirnkarzinom, Leberkarzinom, Lungenkarzinom, Schilddrüsenkarzinom, papilläres Nierenzellkarzinom, Gliom niedrigeren Grads im Gehirn, hepatozelluläres Karzinom, Lungenadenokarzinom und/oder Schilddrüsenkarzinom.

## Revendications

1. Molécule capable de se lier spécifiquement à une fusion de gènes BAIAP2L1:MET, C8orf34:MET, PTPRZ1:MET, OXR1:MET, KIF5B:MET ou TFG:MET, ladite molécule étant un oligonucléotide qui s'hybride à la jonction de fusion d'une fusion de gène MET choisie parmi BAIAP2L1:MET, C8orf34:MET, PTPRZ1:MET, OXR1:MET, KIF5B:MET et TFG:MET
dans laquelle
i) la fusion de gènes BAIAP2L1:MET à laquelle l'oligonucléotide se lie comprend la SEQ ID n° : 1, ou une partie de la SEQ ID n° : 1, ladite partie comprenant la jonction de fusion entre BAIAP2L1 et MET,
ii) la fusion de gènes C8orf34:MET à laquelle l'oligonucléotide se lie comprend la SEQ ID n° : 3, ou une partie de la SEQ ID n° : 3, ladite partie comprenant la jonction de fusion entre C8orf34 et MET,
iii) la fusion de gènes PTPRZ1:MET à laquelle l'oligonucléotide se lie comprend la SEQ ID n° : 5, ou une partie de la SEQ ID n° : 5, ladite partie comprenant la jonction de fusion entre PTPRZ1 et MET,
iv) la fusion de gènes OXR1:MET à laquelle l'oligonucléotide se lie comprend la SEQ ID n° : 7 ou 9, ou une partie de la SEQ ID n° : 7 ou 9, ladite partie comprenant la jonction de fusion entre OXR1 et MET,
v) la fusion de gènes KIF5B:MET à laquelle l'oligonucléotide se lie comprend la SEQ ID n° : 11, ou une partie de la SEQ ID n° : 11, ladite partie comprenant la jonction de fusion entre KIF5B et MET, ou
vi) la fusion de gènes TFG:MET à laquelle l'oligonucléotide se lie comprend la SEQ ID n° : 13, ou une partie de la SEQ ID n° : 13, ladite partie comprenant la jonction de fusion entre TFG et MET.

2. Molécule selon la revendication 1, ladite fusion conduisant à une activité ou une expression aberrante de MET ou à une surexpression de MET.

3. Molécule selon l'une quelconque des revendications 1 à 2, ladite molécule étant un oligonucléotide qui s'hybride spécifiquement à :
a) au moins les nucléotides 951 à 960 de SEQ ID n° : 1 ;
b) au moins les nucléotides 471 à 480 de SEQ ID n° : 3 ;
c) au moins les nucléotides 54 à 63 de SEQ ID n° : 5 ;
d) au moins les nucléotides 62 à 71 de SEQ ID n° : 7 ;
e) au moins les nucléotides 1952 à 1961 de SEQ ID n° : 9 ;
f) au moins les nucléotides 2757 à 2766 de SEQ ID n° : 11 ;
g) au moins les nucléotides 576 à 585 de SEQ ID n° : 13 ; ou
h) un complément de l'un quelconque de a) à g).

4. Méthode de détection de fusion de gène MET dans un échantillon biologique, comprenant la mise en contact d'un échantillon biologique provenant d'un mammifère avec une molécule selon l'une quelconque des revendications 1 à 3, ladite détection de la fusion de gène MET indiquant la présence d'une fusion de MET dans l'échantillon biologique.

5. Méthode de diagnostic d'un cancer chez un patient, la méthode comprenant la mise en contact d'un échantillon biologique provenant du patient avec une molécule selon l'une quelconque des revendications 1 à 3 pour déterminer si une fusion de gène MET est présente dans l'échantillon biologique, ladite détection de la fusion de gène MET indiquant la présence d'un cancer ou une prédisposition à celui-ci.

6. Méthode selon la revendication 5, ledit cancer étant : un carcinome du rein ; un carcinome du cerveau ; un carcinome du foie ; un carcinome du poumon ; un carcinome de la thyroïde ; un carcinome à cellules rénales papillaires ; un gliome du cerveau de grade inférieur, un carcinome hépatocellulaire ; un adénocarcinome pulmonaire ; et/ou un carcinome thyroïdien.
